# EUROPÄISCHE PATENTSCHRIFT

(11) **EP 2 305 034 B1**
(45) Veröffentlichungstag und Bekanntmachung des Hinweises auf die Patenterteilung: **17.04.2013**
(21) Anmeldenummer: 10180601.6
(22) Anmeldetag: 29.05.1999
(51) Int. Cl.: A01N 51/00, A01N 43/36

(54) **Mittel zur Bekämpfung von Pflanzenschädlingen**
Agents for combatting plant pests
Agents pour lutter contre des parasites de végétaux

(30) Priorität: 10.06.1998 DE 19825891; 30.06.1998 DE 19829113
(43) Veröffentlichungstag der Anmeldung: 06.04.2011
(62) Teilanmeldung aus: 05016734.5
(73) Patentinhaber: Bayer CropScience AG, 40789 Monheim (DE)
(72) Erfinder: Erdelen, Christoph, 42799 Leichlingen (DE); Andersch, Wolfram, 51469 Bergisch Gladbach (DE); Stenzel, Klaus, 40595 Düsseldorf (DE); Mauler-Machnik, Astrid, 42799 Leichlingen (DE); Krämer, Wolfgang, 51399 Burscheid (DE)

(56) Entgegenhaltungen:
- WO-A-96/03045
- WO-A1-00/28825

## Beschreibung

Die vorliegenden Erfindung betrifft Schädlingsbekämpfungsmittel die eine Wirkstofikombination der Verbindung der Formel (I) 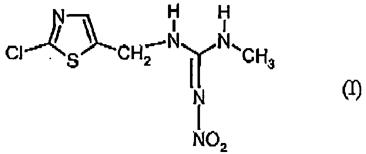 mit Fungiziden enthalten.

Fungizide Wirkstoffe, wie Azol-Derivate, Arylbenzylether, Benzamide, Morpholin-Verbindungen und andere Heterocyclen sind bekannt (vgl. K.H. Büchel "Pflanzenschutz und Schädlingsbekämpfung", Seiten 140 bis 153, Georg Thieme-Verlag, Stuttgart 1977, EP-OS 0 040 345, DE-OS 2 324 010, DE-OS 2 201 063, EP-OS 0 112 284, EP-OS 0 304 758 und DD-PS 140 412).

Mischungen bestimmter Nitromethylenderivate mit fungiziden Wirkstoffen und ihre Verwendung als Schädlingsbekämpfungsmittel im Pflanzenschutz sind bereits bekannt (US-P-4731 385; JP-OS 63-68507, 63/68505; 63/72 608; 63/72 609, 63/72 610, WO 96/03 045, JP 08 245 323, JP 04 368 303, JP 05 017 311, WO 97/22 254, WO 92/21 241). Mischungen bestimmter offenkettigerNitromethylene und Nitroguanidine mit Fungiziden sind bereits bekannt (JP-OS 30 47 106; US-P 5 181 587).

In JP 0410 8704, WO 97/24032, JP 0503 9205, JP 0411 2805 und JP 0412 0007 werden Mischungen der Verbindung der Formel (I) mit anderen Wirkstoffen, darunter auch bestimmten Fungiziden, beschrieben.

Mischungen von Cyclopropylcarboxamiden mit bestimmten Nitromethylenen- oder Nitroguanidinderivaten sind bereits bekannt (JP-OS 3 271 207).

Mischungen von u.a. Imidacloprid und fungiziden Wirkstoffen zur Anwendung im Materialschutz und gegen Termiten, nicht aber zur Anwendung gegen pflanzenschädigende Schädlinge sind bereits bekannt (EP-OS 0 511541). Mischungen von Imidacloprid und Azolyhnethylcycloalkanen insbesondere Triticonazol sind bekannt aus EP-OS 545 834.

Es ist jedoch noch nichts darüber bekannt geworden, dass sich Nitroguanidinderivate und bestimmte Azolderivate in ihrer Wirkung gegenseitig so günstig beeinflussen, daß sie bei guter Pflanzenverträglichkeit in hervorragender Weise als Bekämpfungsmittel gegen Pflanzenschädlinge eingesetzt werden können.

Die vorliegende Erfindung betrifft Mittel gegen Pflanzenschädlinge, die die Verbindung der Fonnel (I) 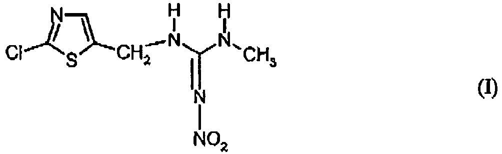 in Mischung mit unten genannten fungiziden Wirkstoffen enthalten.

Als Fungizide in den erfindungsgemäßen Mitteln zur Bekämpfung von Pflanzewchädlingen seien genannt:
(42) Verbindung der Formel

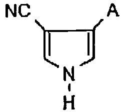

| | | |
|---|---|---|
| A= | 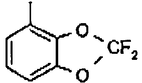 | Fludioxonil |
| A= | 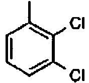 | Fenpiclonil |
| A= | 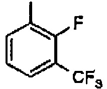 | |

Die erfindungsgemäßen Wirkstoffkombinationen enthalten neben dem Wirkstoff der Formel (I) mindestens einen fungiziden Wirkstoff ausgewählt aus den Verbindungen der Gruppe (1). Sie können darüber hinaus auch weitere Wirkstoffe sowie übliche Hilfs- und Zusatzstoffe sowie Verdümmungsmittel enthalten.

Wenn die Wirkstoffe in den erfindungsgemäßen Wirkstoffkkombinationen in bestimmen Gewichtsverhältnissen vorhanden sind, zeigt sich ein deutlicher synergistischer Effekt der Mischungen. Jedoch können die Gewichtsverhältnisse der Wirkstoffe in den Wirkstoffkombinationen in einem relativ großen Bereich variiert werden. Im allgemeinen entfallen auf 1 Gewichtsteil an Wirkstoff der Formel (I) 0,1 bis 10 Gewichtsteile, vorzugsweise 0,3 bis 3 Gewichtsteile an mindestens einem fungiziden Wirkstoff aus der Gruppe (1).

Die erfindungsgemäßen Wirkstoffkombinationen besitzen sehr gute fungizide Eigenschaften. Sie lassen sich vor allem zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Die erfindungsgemäßen Wirkstoffkombinationen eignen sich besonders gut zur Bekämpfung von Getreidskrankheiten, wie Erysiphe, Cochliobolus, Septoria, Pyrenophora und Leptosphaeria, und gegen Pilzbefall an Gemüse, Wein und Obst, beispielsweise gegen Venturia oder Podosphaera an Äpfeln, Uncinula an Reben oder Sphaeroteca an Gurken.

Die Wirkstoffkombinationen eignen sich auch sehr gut zur Bekämpfung von tierischen Schädlingen, vorzugsweise Arthropoden, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam, Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaca maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp. Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Phylloxera vastatrix, Pemphigus spp., Enpoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopcrtha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Comoderus spp., Melolontha melolontha, Amphimallon solstitialis, Costelytra zcalandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Die gute Pflanzenverträglichkeit der Wirkstoffkombinationen in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungegemäßen Wirkstoffkombinationen können in die üblichen Formulierungen übergeführt werden, wie Lösungen, Emulsionen, Suspensionen, Pulver, Schäume, Pasten. Granulate, Acrosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorothylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, die Dimethylformamid und Dimethylsulfoxid, sowie Wasser. Mit verflüssigten gasförmigen Streckm*itteln oder Trägerstoffen sind solche Flüssigkeiten gemeint, welche bei normaler Temperatur und unter Normaldruck gasförmig sind, z.B. Aerosol-Treibgase, wie Halogonkohlenwasserstoffe, sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomcenerde und synthetische Gesteinsmehle, wie hochdisperse Rieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosuußschaleu, Maiskolben und Tabakstengel. Als Emulgier- und/oder schaummerzeugsende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ethcr, Alkylsulfonate, Alkylsulfate, Arylsulfonate sowie Eiweißhydrolysate. Als Dispergiennittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffkombinationen können in den Formulierungen in Mischung mit anderen bekannten Wirkstoffen vorliegen, wie Fungizide, Insektizide, Akarizide und Herbizide, sowie in Mischungen mit Düngemitteln oder Pflanzenwachtumsregulatoren,

Die Wirkstoffkombinationen können als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver und Granulate, angewendet werden.

Die Anwendung geschieht in üblicher Weise, z.B. durch Gießen, Verspritzen, Versprühen, Verstreuen, Verstrichen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Außerdem wurde gefunden, daß die erfindungsgemäßen Wirksitoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobosirychus brunneus, Sinoxylon spec. Dinoderus minutus.

### Hautflügler wie

Sirex juvencus, Urocerus gigas, Urocerus gigas taignus, Urocenus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverabeitungsprodukte

Unter Holz und Holzverarbeitungsprodukten, welche durch das erfindungsgeinäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdümmungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.%, vorzugsweise 0,001 bis 10 Gew.%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelger misch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaplithalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw, emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.%, verwendet werden. Zusätzlich können an sich bekannte Farbstoffe, Pigmente, wasserabweisende Mittel, Geruchskorrigentien und Inhibitoren bzw. Korrosionsschutzinittel und dgl eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.%, verwendet.

Das erwähnte Bindemittel kann ganz oder teilweise durch ein Pixierungsmittel(gemisch) oder ein Weichumacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Pixierungsmittel basieren chemisch auf Polyvinylalkylethem wie z.B. Polyvinylnethylether oder Ketonen wie Benzophenon, Efhylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide enthalten.

Als zusätzliche Zumischpartner kommen vorzugsweise die in der Wo 94/29 268 genannten Insektizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron genannt.

Die gute pestizide Wirkung der erfindungsgemäßen Wirkstoffkombinationen geht aus den nachfolgenden Beispielen hervor. Während die einzelnen Wirkstoffe oder die bekannten Wirkstoffkombinationen in der pestiziden Wirkung Schwächen aufweisen, geht aus den Tabellen der folgenden Beispiele eindeutig hervor, daß die gefundene Wirkung der erfindungsgemäßen Wirkstoffkombinationen größer ist als Summe der Wirkungen der einzelnen Wirkstoffe und auch größer als die Wirkungen der bekannten Wirkstoffkombinationen.

In den folgenden Beispielen wird der Wirkstoff der Formel (I) 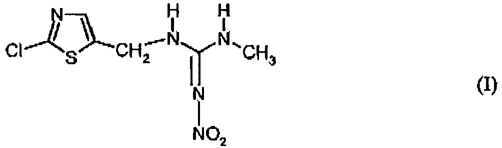 eingesetzt.

Die ebenfalls eingesetzten fungiziden Wirkstoffe werden in den Beispielen angegeben.

## Patentansprüche

1. Mittel, enthaltend die Verbindung der Formel (I) 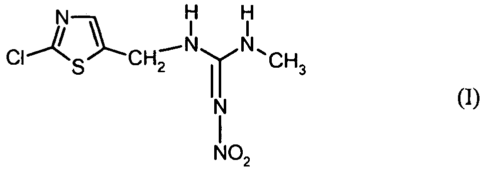 und mindestens eine Verbindung ausgewählt aus
(42) Verbindungen der Formel 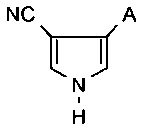
| | | |
|---|---|---|
| A = | 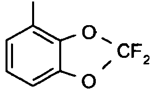 | Fludioxonil |
| A = | 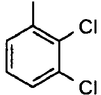 | Fenpiclonil |
| A = | 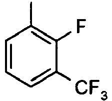 | |

2. Mittel gemäß Anspruch 1, **dadurch gekennzeichnet, dass** in den Wirkstoffkombinationen auf 1 Gewichtsteil an Wirkstoff der Formel (I) 0,1 bis 10 Gew.-Teile an mindestens einem fungiziden Wirkstoff gemäß Formel (42) enfallen.

3. Verfahren zur Bekämpfung von Pilzen und Insekten, **dadurch gekennzeichnet, daß** man Wirkstoffkombinationen gemäß Anspruch 1 oder 2 auf die Pilze, Insekten und/oder deren Lebensraum einwirken läßt.

4. Verwendung von Wirkstoffkombinationen gemäß Anspruch 1 oder 2 zur Bekämpfung von Pilzen und Insekten.

5. Verfahren zur Herstellung von Mitteln gemäß Anspruch 1 oder 2, **dadurch gekennzeichnet, daß** man Wirkstoffkombinationen gemäß Anspruch 1 oder 2 mit Streckmitteln und/oder oberflächenaktiven Stoffen vermischt.

6. Saatgut, welches mit einer effektiven Menge des Mittels gemäß Anspruch 1 oder 2 behandelt ist.

7. Verwendung des Mittels gemäß Anspruch 1 oder 2 zur Behandlung von Saatgut.

## Claims

1. Composition containing the compound of the formula (I) 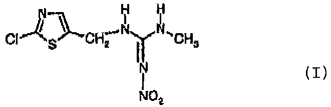 and at least one compound selected from (42) compounds of the formula 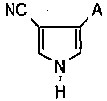
| | | |
|---|---|---|
| A = | 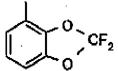 | fludioxonil |
| A = | 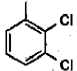 | fenpiclonil |
| A = | 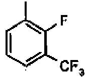 | |

2. Composition according to Claim 1, **characterized in that** the active compound combinations contain from 0.1 to 10 parts by weight of at least one fungicidal active compound according to formula (42) per part by weight of active compound of the formula (I).

3. Process for controlling fungi and insects, **characterized in that** active compound combinations according to Claim 1 or 2 are allowed to act on the fungi, insects and/or their habitat.

4. Use of active compound combinations according to Claim 1 or 2 for controlling fungi and insects.

5. Process for preparing compositions according to Claim 1 or 2, **characterized in that** active compound combinations according to Claim 1 or 2 are mixed with extenders and/or surfactants.

6. Seed treated with an effective amount of the composition according to Claim 1 or 2.

7. Use of the composition according to Claim 1 or 2 for treating seed.

## Revendications

1. Composition contenant le composé de formule (I) 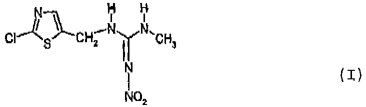 et au moins un composé choisi parmi les composés de formule (42) 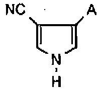
| | | |
|---|---|---|
| A = | 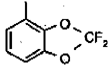 | fludioxonil |
| A = | 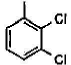 | fenpiclonil |
| A = | 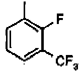 | |

2. Composition selon la revendication 1, **caractérisée en ce que** dans l'association de substances actives pour 1 partie en poids de substance active de formule (I) sont présentes de 0,1 à 10 parties en poids d'au moins une substance active fongicide selon la formule (42).

3. Procédé pour la lutte contre des champignons et des insectes, **caractérisé en ce qu'**on laisse agir les associations de substances actives selon la revendication 1 ou 2 sur les champignons, les insectes et/ou leur habitat.

4. Utilisation d'associations de substances actives selon la revendication 1 ou 2, pour la lutte contre des champignons et des insectes.

5. Procédé pour la préparation de compositions selon la revendication 1 ou 2, **caractérisé en ce qu'**on mélange des associations de substances actives selon la revendication 1 ou 2 avec des diluants et/ou des substances tensioactives.

6. Semence, qui a été traitée par une quantité efficace de la composition selon la revendication 1 ou 2.

7. Utilisation de la composition selon la revendication 1 ou 2 pour le traitement de semences.
